# EUROPÄISCHE PATENTANMELDUNG

(11) **EP 0 255 803 A1**
(43) Veröffentlichungstag der Anmeldung: **10.02.1988**
(21) Anmeldenummer: 87810440.5
(22) Anmeldetag: 31.07.1987
(51) Int. Cl.: A01N 49/00, A61K 9/22, A01N 47/36, A01N 47/34, A01N 47/12, A01N 43/68, A01N 43/32, A01N 43/30, A01N 43/20, A01N 37/38, A01N 35/10

(54) **Verfahren zur Verhinderung des Wiederbefalls von Hunden und Katzen mit Flöhen**

(30) Priorität: 06.08.1986 US 893763
(71) Anmelder: CIBA-GEIGY AG, 4002 Basel (CH)
(72) Erfinder: Barnett, Sharron H., Greensboro, N.C. 27410 (US); Johnson, Roland H., High Point, N.C. 27260 (US); Hepler, Douglas I., High Point, N.C. 27260 (US)

(57) **Zusammenfassung**

Es wird im wesentlichen ein Verfahren beschrieben zur systemischen Verhinderung eines Wiederbefalls von Hunden und Katzen durch Flöhe, dadurch gekennzeichnet, dass man eine für Flöhe larvizid oder ovizid wirksame Menge einer das Flohwachstum hemmenden Substanz oral, parenteral oder in Form eines Implantates dem besagten Wirtstier verabreicht, wobei der zu verabreichende Wirkstoff vorzugsweise
a) ein Juvenil-Hormon oder eine juvenil-hormonartige Substanz,
b) ein das Flohwachstum hemmendes Benzoylharnstoff-Derivat oder
c) ein das Flohwachstum hemmendes Triazinderivat ist.

## Beschreibung

Die vorliegende Erfindung betrifft ein Verfahren zur Eindämmung eines Flohbefalls an Hunden und Katzen. Diese Erfindung betrifft insbesondere ein Floh-Bekämpfungsverfahren, das dadurch charakterisiert ist, dass man eine larvizid oder ovizid wirksame Menge einer über das Blut des Wirtstieres wirkenden, das Wachstum der Flöhe inhibierenden Substanz an den Hund oder die Katze systemisch verabreicht.

Der Befall von blutsaugenden Parasiten an Tieren, insbesondere der Flohbefall an Haus- und Schosstieren bildet immer noch ein nur unzureichend gelöstes Problem für den Veterinär. Auf Grund seines komplizierten Lebenszykluses ist keine der bekannten Methoden zur Bekämpfung von Flöhen rundum zufriedenstellend, insbesondere, weil die bekannten Methoden prinzipiell auf die Bekämpfung der ausgewachsenen Flöhe im Fell ausgerichtet sind und die unterschiedlichen juvenilen Stadien der Flöhe, die nicht nur im Fell des Tieres, sondern auch auf dem Boden, auf Teppichen, auf dem Schlafplatz des Tieres, auf Stühlen, im Garten und all den andern Plätzen, mit denen das befallene Tier in Berührung kommt, existieren, völlig ausser acht lassen. Erwachsene Katzen- und Hundeflöhe (Ctenocephalides felis und C. camis) leben normalerweise im Fell der Wirtskatze bzw. des Wirtshundes. Sie ernähren sich vom Blut des Wirtstieres und legen ihre Eier in sein Fell. Da diese Eier jedoch nicht selbsthaftend sind, fallen sie im allgemeinen rasch ab und können am Boden, auf Teppichen, im Hunde- und Katzenkorb, auf den vom Tier benutzten Stühlen, im Garten, im Hinterhof, usw. gefunden werden.

Das bedeutet, dass der gesamte Lebensraum der Schosstiere mit Floheiern verseucht ist, aus denen sich innerhalb von zwei Tagen die Larven bilden. Man unterscheidet bei den Larven drei Entwicklungsstadien, die jeweils drei Tage dauern. Im letzen Stadium spinnt die Larve ihren Kokon und verwandelt sich in die Puppe. Unter günstigen Bedingungen, d.h. 33°C und einer relativen Luftfeuchtigkeit von 65 %, findet die Umwandlung vom Ei zur Puppe in etwa 8 bis 10 Tagen statt. Nach etwa weiteren 8 Tagen entstehen in den noch immer auf dem Boden, den Teppichen, den Schlafplätzen, den Stühlen, usw. liegenden Kokons die jungen, fertigen Flöhe. Die jungen erwachsenen Flöhe verharren dort bis sie die Gegenwart eines akzeptablen Wirtstieres spüren, dann schlüpfen sie aus ihrem Kokon und versuchen auf das Wirtstier zu springen. Daraus ersieht man, dass es mindestens drei Wochen dauert, bis sich aus einem Ei ein erwachsener Jungfloh entwickelt, der in der Lage ist, das Wirtstier erneut zu befallen.

Dieser Jungfloh kann jedoch monatelang, möglicherweise bis zu einem Jahr in seinem Kokon verbleiben. Andererseits kann unter weniger optimalen Bedingungen die Entwicklung vom Ei bis zum erwachsenen Jungfloh 4 bis 5 Monate beanspruchen. Flöhe benötigen zur Erlangung ihrer sexuellen Reife Blut als Nahrungsmittel, um sich vermehren zu können, und dieses Blut muss auch vom richtigen Wirtstier stammen.

Dieser lange Lebenszyklus, der getrennt vom Wirtstier stattfindet, hat einen bedeutenden Einfluss auf eine erfolgreiche Flohbekämpfung am Wirtstier.

Selbst wenn sich die Flöhe im Fell des Wirtstieres erfolgreich bekämpfen lassen, d.h., wenn alle erwachsenen Flöhe durch eine entsprechende Wirksubstanz abgetötet werden, ist die Katze bzw. der Hund trotzdem für Wochen oder sogar Monate dem Risiko eines Wiederbefalls durch neu ausgeschlüpfte Jungflöhe aus seinem Lebensraum ausgesetzt.

Der Flohbefall von Hunden und Katzen hat nicht nur für das zu behandelnde Tier, sondern auch für den Tierhalter unangenehme Begleiterscheinungen. Derartige Unannehmlichkeiten führen z.B. zu lokalen Reizungen oder lästigem Juckreiz und münden oft zu heftigem Kratzen. Ein grosser Teil der Tiere wird allergisch gegen die Ausscheidungen der Flöhe, was auf dem Tierkörper zu sehr juckenden und krustigen Hautveränderungen rund um die Biss-Stellen führt. Diese Hautveränderungen weisen normalerweise einen Durchmesser von etwa 3 mm oder mehr auf und machen das Tier oft bissig und veranlassen es zu kratzen, so dass es in der Folge stellenweise zu Haarausfall kommt.

Darüberhinaus sind flohinfizierte Tiere dauernd der Gefahr ausgesetzt, dass sie sich mit Dipylidium, einer Bandwurmart, infizieren, die durch Flöhe übertragen wird.

Der Flohbefall ist nicht nur für das befallene Tier äusserst lästig, sondern verursacht auch bei dem Tierhalter unangenehme Begleiterscheinungen, dann schliesslich erkennt dieser an dem ungewöhnlichen Verhalten seines Schosstieres, dass dieses krank ist und leidet und, dass er ihm helfen muss. Darüberhinaus kann es für den Tierhalter unangenehm werden, wenn er sein befallenes Tier abschafft, dieses stirbt oder es zeitweise aus der gewohnten Umgebung entfernt wird, da die auf dem Boden befindlichen neu entpuppten Flöhe bei langer Abwesenheit eines geeigneten Wirtstieres notgedrungenermassen den Menschen befallen, obwohl sie sicht mit menschlichem Blut als einziger Nahrungsquelle nicht vermehren können. Selbst wenn der Hund oder die Katze anwesend ist, kann der Tierhalter von den Flöhen gebissen werden.

Ferner können Hunde- und Katzenflöhe, bzw. deren Ausscheidungen bei manchen Menschen zu Allergie-ähnlichen Hauterkrankungen führen, die in manchen Fällen zur Abschaffung des Schosstieres zwingen. Eine wirksame Bekämpfung der Flöhe bei Hunden und Katzen war daher schon von jeher wünschenswert.

Es sind eine Reihe herkömmlicher Bekämpfungsmethoden bekannt, die jedoch verschiedenartige Nachteile aufweisen. Benützt man z.B. Flohkämme, so bleibt dem Tierhalter nichts anderes übrig, als das Tier intensiv und oft zu kämmen, was je nach Grösse des Tieres wenige Minuten bis zu einer Stunde in Anspruch nehmen kann und nicht von jedem Tier geduldig hingenommen wird. Aber auch nicht jeder Tierhalter ist bereit die Zeit hierfür aufzubringen. Die Verwendung von entsprechenden Floh-aktiven Shampoos kann in vielen Fällen nicht erfolgen, da die meisten Katzen aber auch zahlreiche Hunde sich nicht oder nur unter Gewaltanwendung baden lassen, so das Wasser und Wirkstoff verspritzt werden, die beseitigt werden müssen. Ausserdem hält die Wirkung einer solchen Bade-Behandlung höchstens ca. eine Woche lang an, und die umständliche Prozedur muss wiederholt werden. Mit den selben oder ganz ähnlichen Problemen hat man bei der Verwendung von Einölungen (dips) oder Spülungen (rinses) zu rechnen. Auch der Einsatz von Pudern (dusting powders) wird vom Tier im allgemeinen nicht widerstandslos hingenommen, da man doch einige Minuten braucht, und die gesamte Felloberfläche gleichmässig zu behandeln, wobei zwangsläufig etwas Staub in Maul, Nase und Augen gelangt. Selbst bei sorgfältiger Anwendung kann nicht ausgeschlossen werden, dass Tier und Mensch von dem Puder inhalieren. Es ist praktisch unvermeidbar, dass auch der Mensch in mehr oder weniger intensiven Kontakt mit dem Mittel kommt.

Bei dem Einsatz von Sprays kann manch einer die unliebsame Ueberraschung erleben, da die meisten Tiere insbesondere Katzen, bereits bei dem Sprühgeräusch die Flucht ergreifen oder aggressiv reagieren. Darüberhinaus haben Sprays auch alle unter den Pudern aufgezählten Nachteile, wobei hinzukommt, dass sie sich noch feiner in der Atmosphäre verteilen und daher von Mensch und Tier inhaliert werden. Flöhe werden häufig auch mit sogenannten Floh-Halsbändern bekämpft, die vorübergehend eine gute Effektivität gewährleisten. Eine gewisse Schwäche zeigt sich bei dieser Behandlung insbesondere durch die lokal sehr begrenzte Applikation. Zwar beträgt die abtötende Wirkung im Hals- und Brustbereich im allgemeinen 100 %; weiter weg liegende Körperpartien wurden jedoch kaum beeinflusst. Ausserdem ist die Wirkung dieser Bänder zeitlich begrenzt. Daneben sehen viele dieser Halsbänder unattraktiv aus und können das Tier stören. Man kann heute auch Medaillons kaufen, die an gewöhnliche Halsbänder gehängt werden können und aktiv sein sollen. Diese sehen zwar optisch ansprechend aus, ihre Wirkung ist jedoch unbefriedigend, da der Fellkontakt mangelhaft ist. Einige floh-aktive Organophosphorverbindungen werden auch als Spot-On-Formulierungen angeboten und somit auf einer lokal begrenzten Stelle des Fells aufgetragen. Sie zeigen im allgemeinen gegen adulte Flöhe kurzfristig gute Wirkung, wobei die angewendeten Mittel oft jedoch problematische Toxwerte aufweisen. Organophosphorverbindungen wurden zum Teil auch oral verabreicht, wobei ihnen jedoch enge Sicherheitsgrenzen gesetzt sind und die keinesfalls mit anderen Organophosphorverbindungen gleichzeitig appliziert werden dürfen.

Insgesamt kann gesagt werden, dass die bisherigen Verfahren stets die Abtötung des adulten Flohs anstrebten und diesen zum Teil durchaus kurzfristig gut bekämpfen. Was jedoch bisher nicht erkannt wurde, ist die Tatsache, dass auf Grund des besonderen Lebenszyklus der Flöhe Hunde und Katzen immer wieder von neuen infiziert werden, zum einen, da ein Kontakt mit den Floheiern, Flohlarven und jungen, adulten Flöhen auf dem Boden bzw. in der nächsten Umgebung des Tieres micht vermeidbar ist, zum anderen weil viele Schosstiere immer wieder mit infizierten Artgenossen in Berührung kommen. Die ständig wiederkehrende Reinfestation wird mit herkömmlichen Mitteln nicht verhindert.

Es wurde nun überrascgenderweise festgestellt, dass man mit Hilfe bestimmter Applikationsarten und unter Einsatz von Aktivsubstanzen, die die juvenilen Flohstadien in ihrer Entwicklung blockieren, den weiter oben ausführlich dargestellten Teufelskreis der ständig wiederkehrenden Reinfestation auf einfache Weise durchbrechen kann, somit die Flöhe an ihrer Vermehrung hindert und darüberhinaus den Lebensraum von Hunden und Katzen auf Dauer flohfrei halten kann.

Es wurde nämlich gefunden, dass durch orale Gabe, parenterale Gabe oder auch durch Implantat einer larvizid oder ovizid wirksamen Menge einer das Flohwachstum hemmenden Substanz der Wiederbefall durch Flöhe an Katzen und Hunden drastisch reduziert oder völlig verhindert werden kann.

Es wurde ferner gefunden, dass sich für diesen Einsatz insbesondere folgende drei Klassen von Aktivsubstanzen eignen, nämlich:
a) ein Juvenil-Hormon oder eine Juvenil-hormon-artige Substanz,
b) ein das Flohwachstum inhibierendes Benzoylharnstoff-Derivat oder
c) ein das Flohwachstum inhibierendes Triazin-Derivat.

Zwar war schon von einigen Vertretern aus den Substanzklassen (a), (b) und (c) bekannt, dass sie die Weiterentwicklung von juvenilen Entwicklungsstadien hemmen bzw. vollständig unterdrücken; diese Wirkung wurde jedoch stets bei der direkten Applikation des Wirkstoffs auf den Parasiten festgestellt. Erstaunlich im Zusammenhang mit der vorliegenden Erfindung ist jedoch, dass die volle Wirkung auch dann noch erreicht wird, wenn man den Wirkstoff in relativ niedrigen Aufwandmengen an das Wirtstier verabreicht und er erst auf dem Umweg über das aufgesaugte Blut den adulten Floh und zusätzlich über die Kotaufnahme die Flohlarve, das eigentliche Zielobjekt erreicht. Mit der Abtötung der adulten Flöhe, was bisher stets das Ziel war, ist keine Ausmerzung de Flöhe erreichbar. Es wurde vielmehr gefunden, dass eine effektive Flohbekämpfung nur dann gewährleistet ist, wenn man erfolgreich verhindert, dass aus den Floheiern neue Flohpopulationen entstehen.

Die zu bekämpfenden Seuchen können auf zwei Arten von den Substanzen erreicht werden. Chemikalien können über das Blutmahl vom erwachsenen Floh aufgenommen und vom erwachsenen Floh an die Eier weitergegeben werden, wo sie dann ihre Wirkung entfalten. Der Tod kann dann im Ei-, Larven- oder Puppenstadium eintreten. Untersuchungen an Flöhen zeigen eine reduzierte Eiablage, hohe Mortalität der Larven und eine Hemmung der Schlupffähigkeit im Falle des Puppenstadiums. Zusätzlich zur Wirkung auf die Eier, sind die Flohlarven dem Einfluss der Ausscheidungen der erwachsenen Flöhe ausgesetzt, da sie sich von diesen Exkrementen ernähren. Die Ausscheidungen von Flöhen enthalten noch hohe Anteile an unverdautem Blut des Wirtstieres und dienen den sich entwickelnden Flöhen als Proteinquelle.

Die vorliegende Erfindung umfasst somit zwei Aspekte, einmal die bereits beschriebene Methode zur Verhinderung einer Reinfestation von Hunden und Katzen mit Flöhen, gleichzeitig natürlich auch die Unterdrückung der Vermehrung von Flöhen oder genauer ausgedrückt: Ein Verfahren zur Unterdrückung der Fortpflanzung von Flöhen, das dadurch gekennzeichnet ist, dass man den Flöhen als Nahrungsmittel Blut verabreicht, das eine wirksame Menge mindestens einer der weiter oben definierten Wirksubstanzen enthält. Dieses Verfahren schliesst auch den Aspekt ein, dass man eine wirksame Menge einer der besagten Wirksubstanzen an die Wirtskatze oder den Wirtshund im Futter verabreicht und die Flöhe durch Aufnahme des tierischen Blutes beim Blutsaugen die Wirksubstanz einnehmen.

Alle drei Wirksubstanzklassen (a), (b) und (c) einschliesslich ihrer Herstellung werden in der Literatur beschrieben. So werden Juvenil-Hormone bzw. Substanzen, die wie Juvenil Hormone wirken (Klasse a) in folgenden Publikationen beschrieben: Chemie der Pflanzenschutz- und Schädlingsbekämpfungsmittel, R. Wegler, Springer-Verlag Berlin, Heidelberg, New York (1981), Vol. 6, S. 185 bis 214.

Insecticide Mode of Action, Joel R. Coats, Academic Press (1982) S. 315 bis 402.
US-PS-4,172,146; US-PS-3,987,102; US-PS-3,987,108; US-PS-3,988,477; US-PS-4,007,280; US-PS-4,100,296; US-PS-4,097,581; US-PS-4,060,629; US-PS-4,080,470; EP-4,334; UK-2,084,574; EP-169,169; EP-72,475; US-PS-4,092,365; US-PS-4,057,587; US-PS-4,017,549; DE-OS-2,528,345; EP-37,092; US-PS-4,061,683; DE-OS-2,925,113; Chem.Abstr., Vol. 102, S. 668, 113025e; Chem.Abstr., Vol. 102, S. 222, 57842e; Chem.Abstr., Vol. 102, S. 569, 61919c; UK-2,102,425, und Agric.Biol.Chem., Vol. 49, S. 3197 bis 3202 (1985).

Jeder der nachfolgend mit Formel wiedergegebene Vertreter wird als besonders bevorzugte Substanz der Klasse (a) genannt:

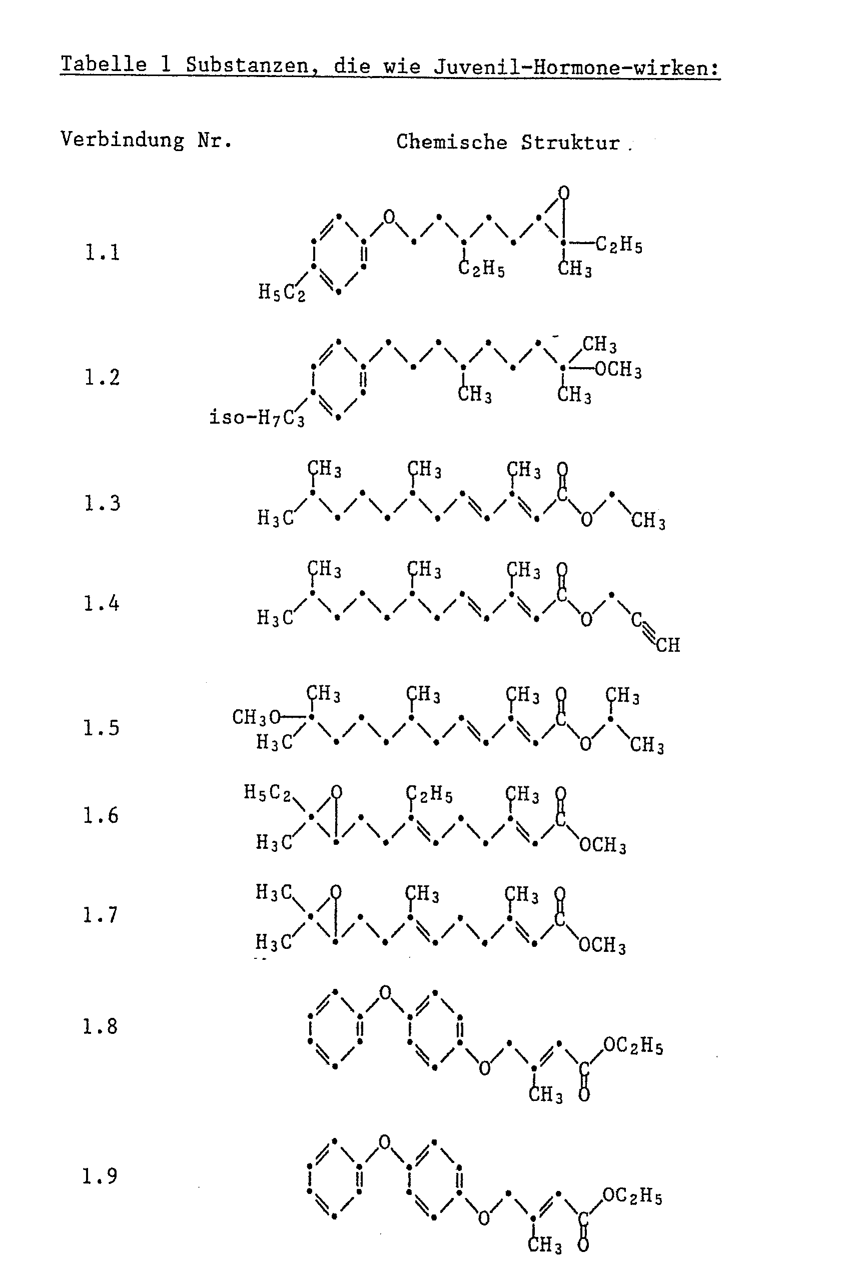

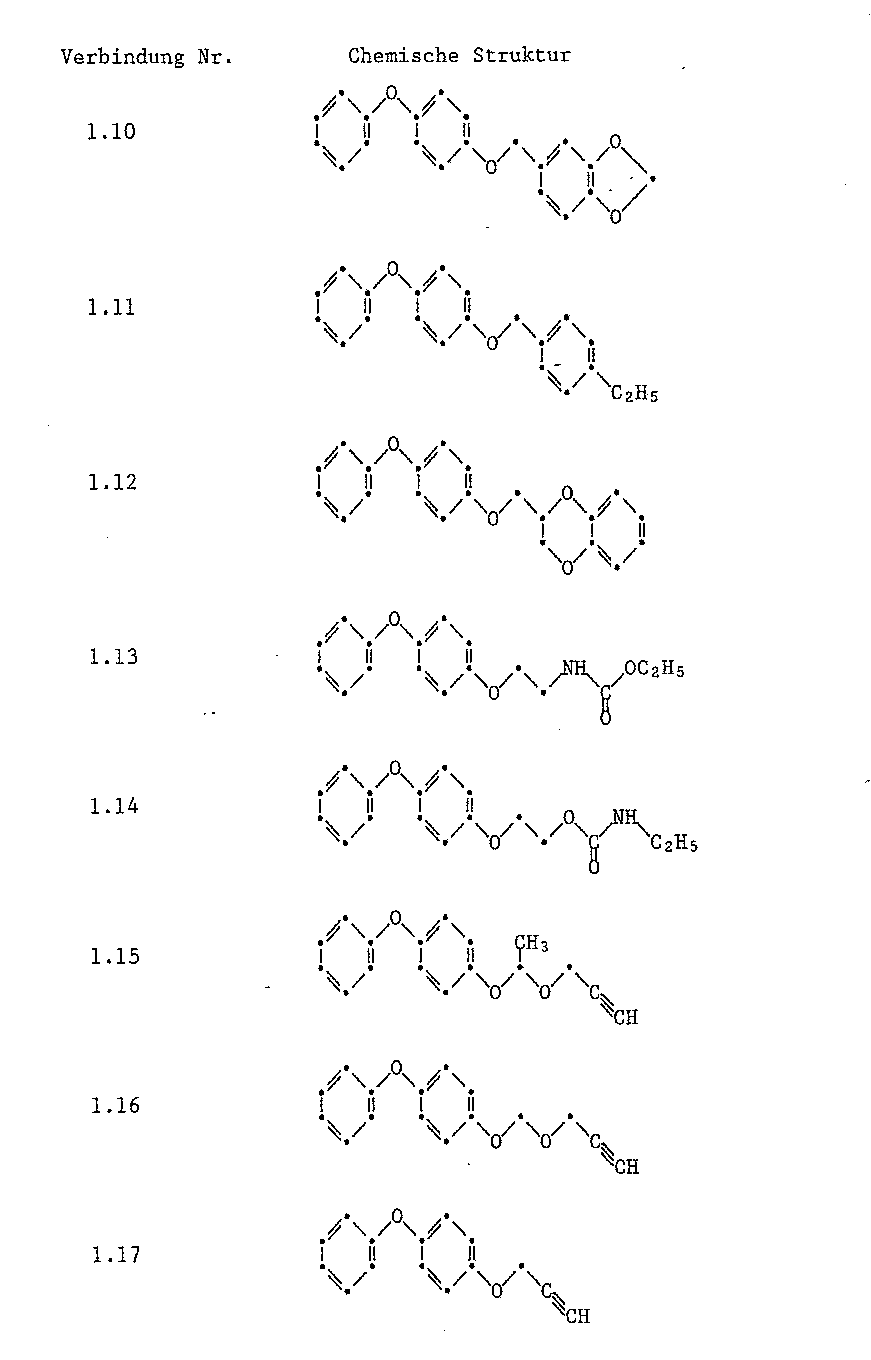

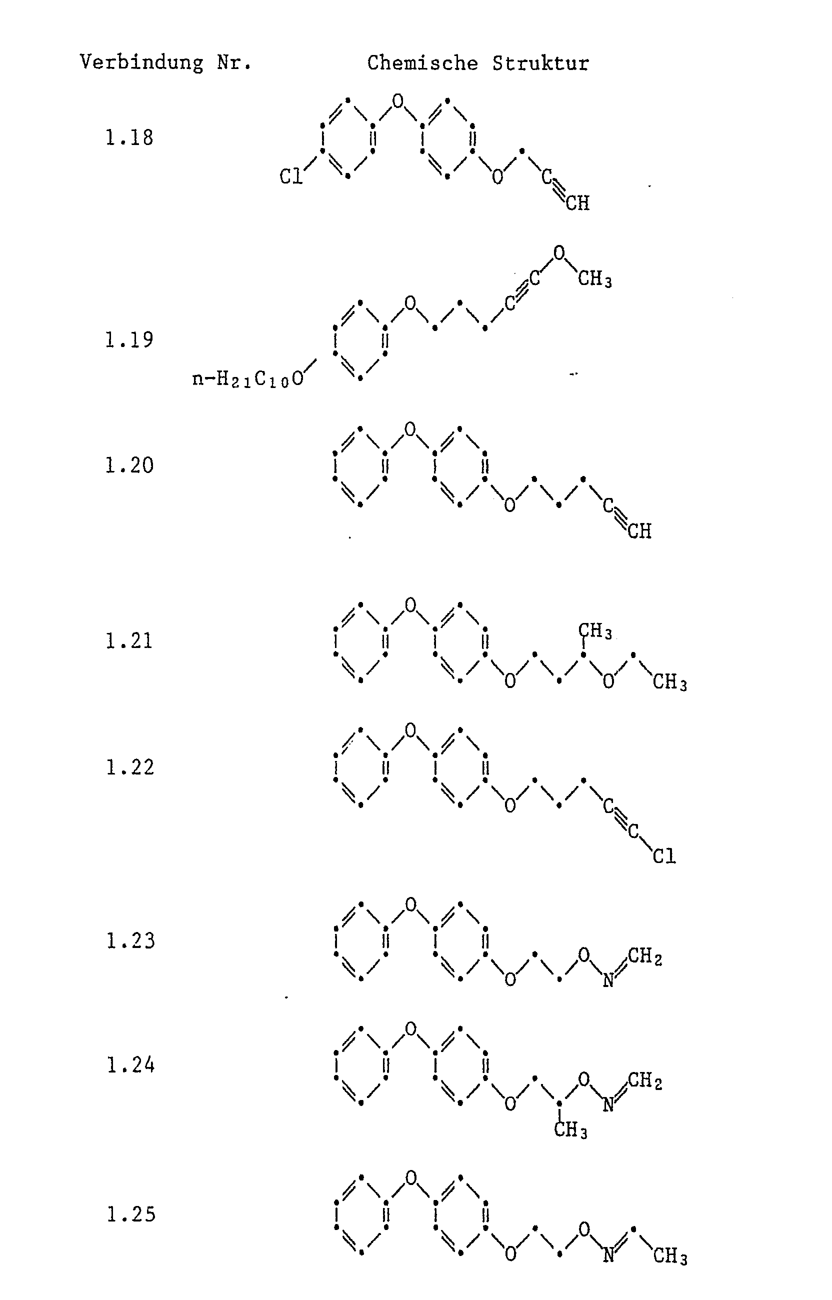

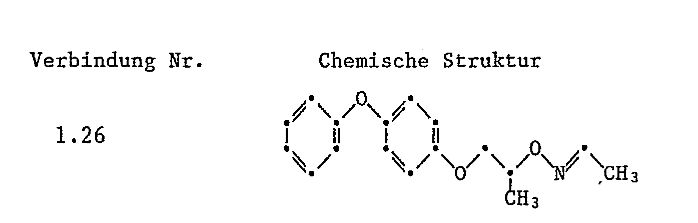

Klasse (b): Benzoylharnstoff-Derivate, die das Wachstum von Flöhen hemmen und auch unter der Bezeichnung "Dimiloide" bekannt sind, werden z.B. in folgender Literatur beschrieben: DE-OS-2,123,236; DE-OS-2,601,780; DE-OS-3,240,975; EP-72,438; EP-42,533 und insbesondere EP-79,311.

Bei den für den erfindungsgemässen Einsatz in Frage kommenden Verbindungen der Klasse (b) handelt es sich um folgende Verbindungen der Formel I 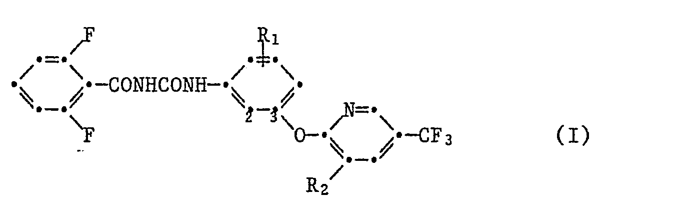 worin
R₁ für Wasserstoff, Halogen oder Methyl steht; und
R₂ Wasserstoff oder Halogen bedeutet.

Eine bevorzugte Untergruppe bilden Verbindungen der Formel I, worin
R₁ für Wasserstoff, Fluor, Chlor oder Brom steht; und
R₂ Wasserstoff, Fluor, Chlor oder Brom bedeutet.

Besonders bevorzugte Vertreter der Formel I sind solche, worin
R₁ für 4-Fluor, 4-Chlor, 4-Brom oder 4-Methyl steht;
und
R₂ Chlor bedeutet.

Als besonders bevorzugte Einzelvertreter von Verbindungen der Formel I [Klasse (b)] werden genannt:

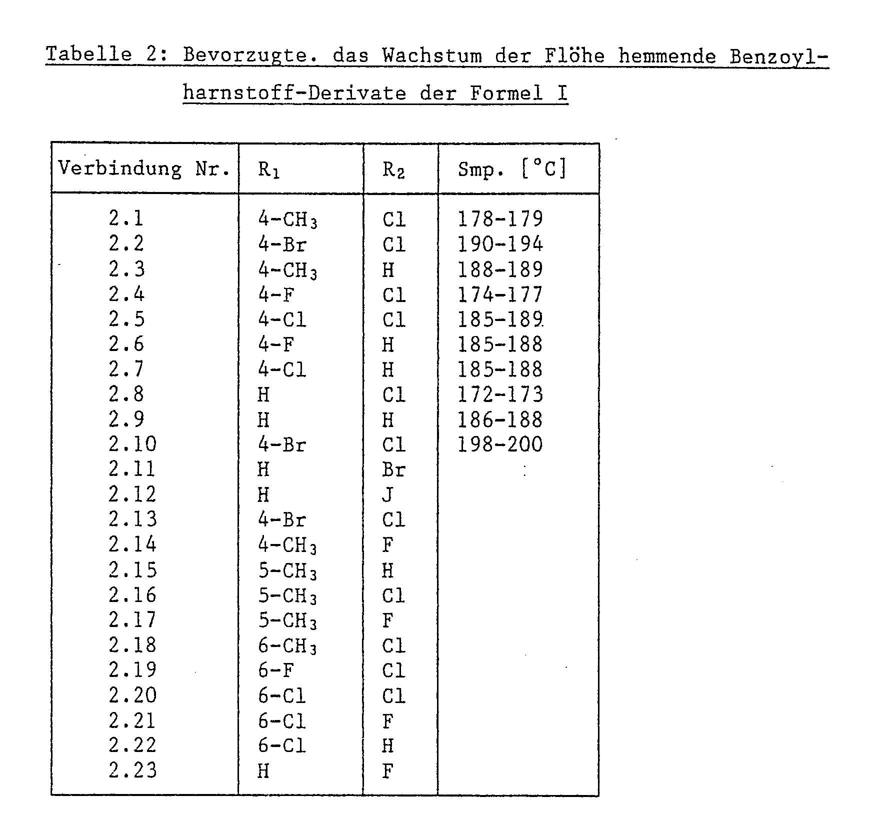

Klasse (c): Erfindungsgemäss verwendbare, das Wachstum der Flöhe hemmende Triazin-Derivate werden beispielsweise beschrieben in: US-PS-4,225,598; US-PS-4,160,831; US-PS-4,160,832; US-PS-4,187,304; US-PS-4,187,305 und DE-OS-3,030,646.

Folgende Gruppe von Verbindungen dieser Klasse (c) ist als besonders geeignet hervorzuheben:
Verbindungen der Formel X 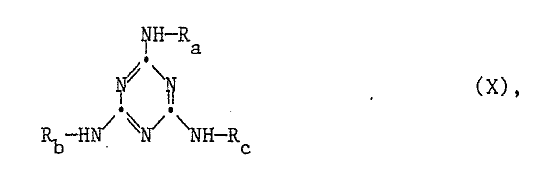 worin
Rₐ für Cyclopropyl oder Isopropyl steht;
R_{b} Wasserstoff, Halogen, C₁-C₁₂-Alkylcarbonyl, Cyclopropylcarbonyl, C₁-C₁₂-Alkylcarbamoyl, C₁-C₁₂-Alkylthiocarbamoyl oder C₂-C₆-Alkenylcarbamoyl bedeutet;
und
R_{c} für Wasserstoff, C₁-C₁₂-Alkyl, Cyclopropyl, C₂-C₆-Alkenyl, C₁-C₁₂-Alkylcarbonyl, Cyclopropylcarbonyl, C₁-C₁₂-Alkylcarbamoyl, C₁-C₁₂-Alkylthiocarbamoyl oder C₂-C₆-Alklenylcarbamoyl steht und deren Säureadditionssalze, die für Warmblüter untoxisch sind.

Die nachfolgende Tabelle 3 zeigt typische Vertreter von Verbindungen der Formel X

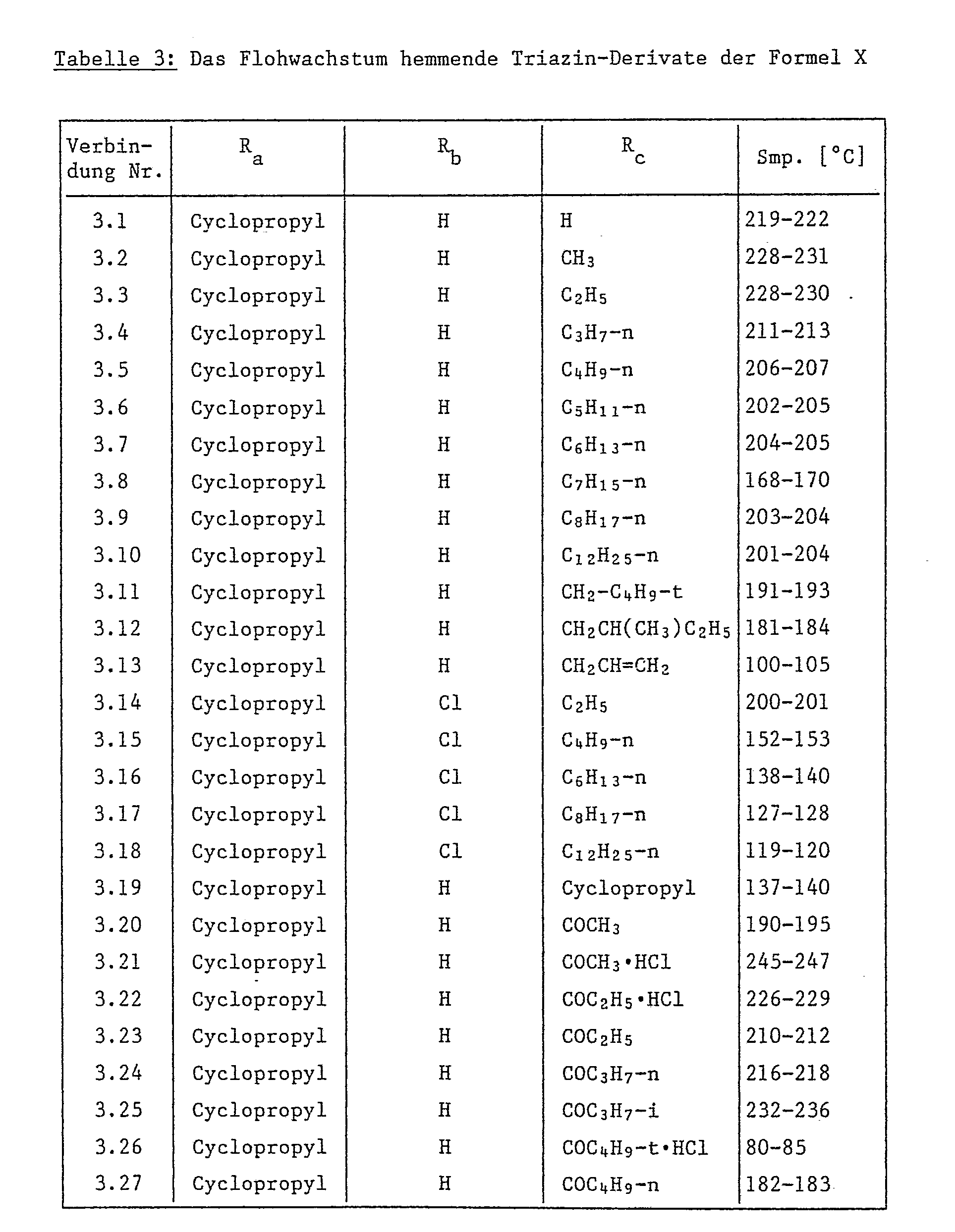

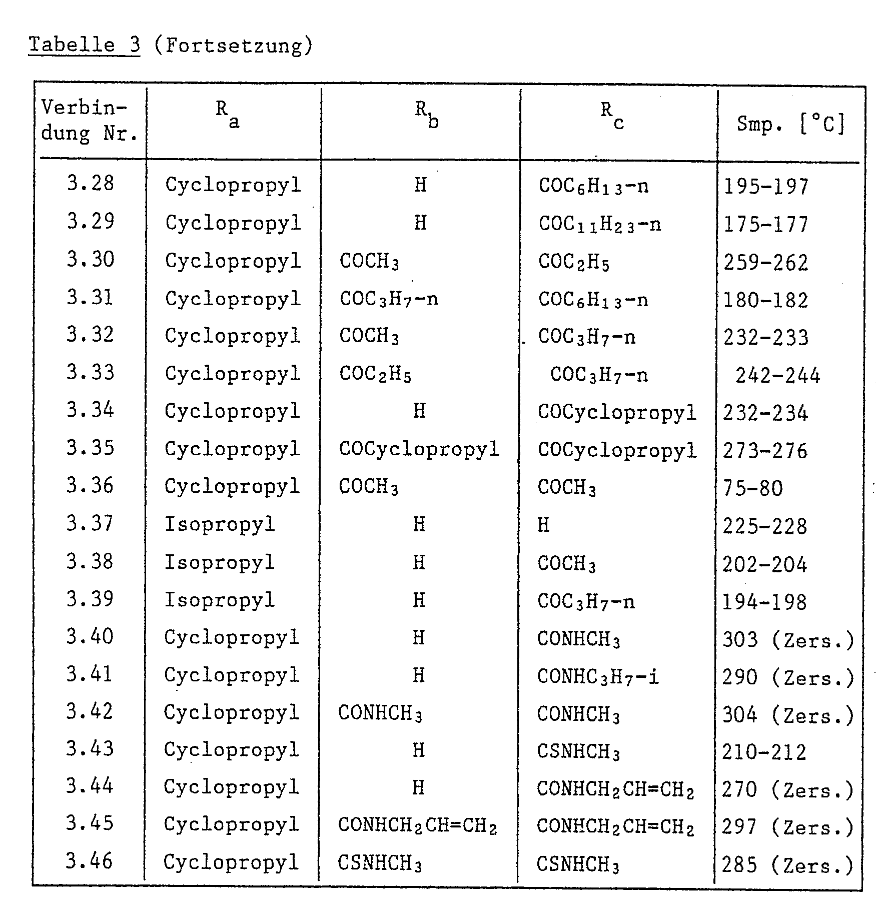

Besonders bevorzugt ist die Verbindung 3.1 (Cyromazin)

Erfindungswesentlich ist, dass der aus den genannten Substanzklassen (a), (b) oder (c) ausgewählte Wirkstoff so verabreicht wird, dass er vom adulten, saugenden Floh mit dem Blut des Wirtstieres aufgenommen werden und in den juvenilen Flohstadien zur Wirkung gelangen kann. Dies wird erfindungsgemäss mit verschiedenen Applikationsformen erreicht, z.B. indem man einen formulierten Wirkstoff oral verabreicht. Formuliert heisst in diesem Fall z.B. in Form eines Pulvers, einer Tablette, eines Granulats, einer Kapsel, einer Emulsion, eines Schaumes etc., wobei man das Präparat dem Tier nicht unbedingt direkt zu geben braucht, sondern zweckmässigerweise unter sein Futter mischt. Selbstverständlich können alle oral zu verabreichenden Kompositionen neben üblichen Formulierungsstoffen weitere Zusätze enthalten, die die freiwillige Aufnahme durch das Tier fördern, z.B. geeignete Duft- und Aromastoffe. Die orale Verwendung ist, auf Grund ihrer einfachen Ausführbarkeit einer der bevorzugten Gegenstände dieser Erfindung. Eine weitere Applikationsart ist der parenterale Einsatz, z.B. durch subkutane Injektion oder Injektion in die Vene oder das Langzeitpräparat (Depotform) in Form eines Implantates.

Die orale Applikation schliesst z.B. das Verabreichen von Hunde- und Katzenfutter ein, das die Aktivsubstanz bereits beigemischt enthält, z.B. als Biskuits oder als Leckerbissen, als Kautablette, als wasserlösliche Kapseln oder Tabletten, in wasserlöslicher Form die auf das Futter getropft werden kann oder in sonstigen, dem Tierfutter beimengbaren Formen. Die Implantate schliessen auch alle Vorrichtungen ein, die in den Körper des Tieres zur Substanzabgabe eingebracht werden können.

Perkutane Applikationsformen schliessen beispielsweise die subkutane, dermale, intramuskuläre und sogar intravenöse Verabreichung injizierbarer Formen ein. Dabei können ausser den üblichen Injektionsspritzen mit Nadeln auch nadellose Druckstossapparate, aber auch Pour-on- und Spot-on-Formulierungen zweckdienlich sein.

Durch Wahl einer geeigneten Formulierung ist es möglich das Eindringungsvermögen des Wirkstoffes durch das lebende Gewebe des Tieres zu fördern bzw. seine Verfügbarkeit aufrechtzuerhalten. Dies ist von Bedeutung, wenn z.B. ein sehr schwerlöslicher Wirkstoff eingesetzt wird, dessen geringe Löslichkeit eine löslichkeitsfördernde Massnahme erfordert, da die Körperflüssigkeit des Tieres nur geringe Mengen des Wirkstoffes auf einmal zu lösen vermag.

Ferner kann der Wirkstoff auch in einer Matrixformulierung vorliegen, die auf physikalische Weise seine Zersetzung verhindert und die laufende Verfügbarkeit des Wirkstoffs aufrechterhält. Diese Matrixformulierung wird in den Körper injiziert und verbleibt dort als eine Art Depot, aus dem der Wirkstoff kontinuierlich freigesetzt wird. Derartige Matrixformulierungen sind dem Fachmann bekannt.Es handelt sich im allgemeinen um wachsartige, halbfeste Substanzen, wie z.B. pflanzliche Wachse und Polyethylenglykole mit hohem Molekulargewicht.

Eine hohe Verfügbarkeit des Wirkstoffs wird auch durch Einführung eines Implantats der Aktivsubstanz in das Tier erhalten. Solche Implantate sind in der Veterinärmedizin weit verbreitet und bestehen oftmals aus silikonhaltigem Gummi. Die Aktivsubstanz ist dabei im festen Gummi dispergiert oder befindet sich im Innern eines Gummihohlkörpers. Zu beachten ist dabei, dass eine Aktivsubstanz ausgewählt wird, die im Gummiimplantat löslich ist, da sie zuerst im Gummi gelöst wird und dann kontinuierlich aus dem Gummimaterial in die Körperflüssigkeit des zu behandelnden Tieres sickert.

Die Freigaberate der Aktivsubstanz aus dem Implantat und damit die Zeitspanne, in der das Implantat eine Wirkung zeigt, wird im allgemeinen von der Genauigkeit der Eichung (Wirkstoffmenge im Implantat) des Implantats, der Umgebung des Implantats und der Polymerformulierung aus der das Implantat hergestellt ist, bestimmt.

Die Verabreichung des Wirkstoffs mittels Implantat stellt einen weiteren bevorzugten Bestandteil der vorliegenden Erfindung dar. Eine derartige Verabreichung ist äusserst ökonomisch und effektiv, weil ein richtig dimensioniertes Implantat eine konstante Konzentration der Wirksubstanz im Gewebe des Wirtstieres gewährleistet. Implantate können heutzutage derart gestaltet und in einfacher Weise implantiert werden, dass sie den Wirkstoff über einige Monate zu liefern im Stande sind. Nach dem Implantieren entfällt jede weitere Belästigung des Tieres, und man braucht sich auch keinerlei Sorgen wegen der Dosierung mehr machen.

Die Verabreichung von veterinärmedizinischen Zusätzen zum Tierfutter ist auf dem Gebiet der Tiergesundheit bestens bekannt. Ueblicherweise wird zunächst ein sogenanntes Premix (Vormixtur) hergestellt, in dem die Aktivsubstanz in einer Flüssigkeit dispergiert oder feinverteilt in festen Trägermaterialien vorliegt. Dieses Premix kann normalerweise, in Abhängigkeit von der gewünschten Endkonzentration im Futter, etwa 1 bis 800 g der Substanz pro kg Premix enthalten.

Es ist zudem bekannt, dass Wirkstoffe durch die Bestandteile des Futters hydrolisiert oder geschwächt werden können. Solche Wirksubstanzen werden routinemässig in einer Schutzmatrix, z.B. in Gelatine, formuliert, bevor man sie dem Premix zusetzt.

Folglich betrifft die vorliegende Erfindung auch ein Verfahren zur systemischen Vorbeugung einer Reinfektion von Hunden und Katzen durch Flöhe, das sich dadurch kennzeichnet, dass man dem besagten Wirtstier oral, parenteral oder mittels eines Implantates eine larvizid oder ovizid wirksame Menge eier das Wachstum der Flöhe hemmenden Substanz zusetzt, vorzugsweise ein Verfahren, das sich dadurch auszeichnet, dass man als eine das Wachstum der Flöhe hemmende Substanz eine Verbindung aus der Gruppe:
a) Juvenil Hormon oder juvenil-hormon-artige Substanz,
b) das Flohwachstum hemmendes Benzoylharnstoffderivat, oder
c) das Flohwachstum hemmendes Triazin-Derivat.

Die vorliegende Erfindung betrifft somit auch ein Verfahren zur Verhütung der Vermehrung von Flöhen, das sich dadurch kennzeichnet, dass man Flöhen als Nahrungsmittel Blut zur Verfügung stellt, das eine wirksame Menge einer das Flohwachstum hemmenden Substanz aus einer der weiter oben beschriebenen drei Stoffklassen enthält. Anders ausgedrückt, betrifft die vorliegende Erfindung auch ein Verfahren zur Verhütung der Vermehrung von auf Hunden und Katzen befindlichen Flöhen, das sich dadurch kennzeichnet, dass man eine wirksame Menge einer der weiter oben charakterisierten, das Floh wachstum hemmenden Substanzen, dem Wirtstier in Form von Futterzusatz verabreicht und auf diese Weise an die auf dem Wirtstier lebenden Flöhe gelangen lässt.

Im Rahmen der vorliegenden Erfindung wird unter einer juvenil-hormon-ähnlichen bzw. juvenil-hormon-artigen Substanz, eie chemische Verbindung verstanden, die die Eigenschaften eines Juvenil-hormons aufweist bzw. wie ein solches wirkt.

Der Wirkstoff aus einer der Klassen (a), (b) oder (c) wird zweckmässigerweise in einer Dosierung von 0,01 bis 800, vorzugsweise 0,5 bis 200, insbesonders 1 bis 30 mg/kg Körpergewicht in Bezug auf das Wirtstier appliziert, wobei die orale Verabreichung bevorzugt ist.

Eine gute Dosis, die regelmässig an das Wirtstier verabreicht werden kann, liegt 1 bis 100 mg/kg Körpergewicht. Die Verabreichung erfolgt zweckmässigerweise täglich oder wöchentlich.

Die Gesamtdosis kann bei gleichem Wirkstoff von einer Tiergattung zur anderen und auch innerhalb einer Tiergattung variieren, da sie u.a. vom Gewicht und der Konstitution des Tieres abhängt.

Bei der erfindungsgemässen Verwendung wird der Wirkstoff normalerweise nicht in reiner Form appliziert, sondern vorzugsweise in Form eines Mittels, das neben dem Wirkstoff applikationsfördernde Bestandteile enthält, wobei solche Bestandteile in Frage kommen, die dem Wirtstier zuträglich sind. Selbstverständlich können neben der erfindungsgemässen Bekämpfung der juvenilen Entwicklungsstadien zusätzlich mit konventionellen Methoden die adulten Flöhe bekämpft werden, letzteres ist jedoch nicht zwingend notwendig.

Derartige erfindungsgemäss anzuwendende Mittel enthalten in der Regel 0,1 bis 99 Gew.-%, insbesonders 0,1 bis 95 Gew.-%, Wirkstoff aus einer der Klassen (a), (b) oder (c), und 99,9 bis 1 Gew.-%, insbesondere 99,9 bis 5 Gew.-%, eines festen oder flüssigen, untoxischen Zusatzstoffes, darunter 0 bis 25 Gew.-%, insbesondere 0,1 bis 25 Gew.-%, eines untoxischen Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Als Formulierungshilfsstoffe können die aus der veterinärmedizinischen Praxis für orale, parenterale und Implantate bekannten Materialien eingesetzt werden. Nachfolgend seien einige Beispiele genannt.

Geeignete Tragerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten, Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe, Aromastoffe oder Pigmente, z.B. zur Identifizierung oder zur Kennzeichnung verschiedener Wirkstoffdosen, beigefügt werden.

Weitere oral anwendbare Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können. Bevorzugt sind u.a. Kapseln, die sowohl leicht zerbissen als auch unzerkaut geschluckt werden können.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: Tabletten enthaltend 25 mg des Wirkstoffs, z.B. 2-Cyclopropylamino-4,6-bis(dimethylamino)-s-triazin, können folgendermassen hergestellt werden:

Bestandteile (für 1000 Tabletten)
Wirkstoff 25,0 g
Lactose 100,7 g
Weizenstärke 7,5 g
Polyethylenglykol 6000 5,0 g
Talkum 5,0 g
Magnesiumstearat 1,8 g
entmineralisiertes Wasser q.s.

Herstellung: Sämtliche festen Ingredienzien werden zunächst durch ein Sieb mit 0,6 mm Maschenweite getrieben. Dann wird der Wirkstoff die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Maschenweite verpresst.

Beispiel 2: Tabletten enthaltend 0,02 g Wirkstoff, z.B. das 2-Cyclopropylamino-4,6-bis(dimethylamino)-s-triazin werden wie folgt hergestellt:

Zusammensetzung (für 10'000 Tabletten)
Wirkstoff 200,00 g
Lactose 290,80 g
Kartoffelstärke 274,70 g
Stearinsäure 10,00 g
Talk 200,00 g
Magnesiumstearat 2,50 g
Kolloidales Siliciumdioxid 32,00.
Ethanol q.s.

Ein Gemisch des Wirkstoffs, der Lactose und 194,70 g Kartoffelstärke wird mit einer ethanolischen Lösung der Stearinsäure befeuchtet und durch ein Sieb granuliert. Nach dem Trocken mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen werden können.

Beispiel 3: Kapseln, enthaltend 0,025 g des Wirkstoffs, z.B. das 2-Cyclopropylamino-4,6-bis(dimethylamino)-s-triazin können wie folgt hergestellt werden:

Zusammensetzung (für 1000 Kapseln)
Wirkstoff 25,00 g
Lactose 249,80 g
Gelatine 2,00 g
Maisstärke 10,00 g
Talk 15,00 g
Wasser q.s.

Man mischt den Wirkstoff mit der Lactose, befeuchtet die Mischung gleichmässig mit einer wässrigen Lösung der Gelatine und granuliert sie durch ein Sieb mit einer Maschenweite von 1,2-1,5 mm. Das Granulat mischt man mit der getrockneten Maisstärke und dem Talk und füllt Portionen von 300 mg in Hartgelatinekapseln (Grösse 1) ab.

### Beispeil 4: Premix (Futterzusatzmittel)

0,25 Gewichtsteile Wirkstoff und
4,75 Gewichtsteile sekundäres Calciumphosphat, Tonerde, Aerosil, Carbonat oder Kalk werden bis zur Homogenität mit
95 Gewichtsteilen eines Tierfutters gemischt.

### Beispiel 5: Injizierbare Lösung

8 Gewichtsteile Wirkstoff,
3,6 Gewichtsteile Essigsäure,
88,4 Gewichtsteile von Wasser für die Injektion.
Die Essigsäure und das Wasser werden gemischt und zum Wirkstoff gegeben. Das Gemisch wird nun so lange gerührt, bis sich alles gelöst hat. Die Lösung wird anschliessend filtriert und nach an sich bekannten Methoden sterilisiert. Der pH-Wert der Lösung liegt bei 5,0.

### Beispiel 6: Emulgierkonzentrat

20 Gewichtsteile des Wirkstoffs werden mit
20 Gewichtsteilen des Emulgators, z.B. eines Gemisches von Alkylarylpolyglykolether mit Alkylarylpolysulfonaten, und mit
60 Gewichtsteilen eies Lösungsmittels so lange gemischt, bis die Lösung vollständig homogenisiert ist. Durch Verdünnen mit Wasser erhält man Emulsionen gewünschter Konzentration.

### Beispiel 7: Lösungen (z.B. zur Verwendung als Trinkzusatz)

15 Gewichtsprozent des Wirkstoffs in 2,2-Dimethyl-4-hydroxymethyl-1,3-dioxolan,
10 Gewichtsprozent des Wirkstoffs in Diethylenglykolmonoethylether,
10 Gewichtsprozent in Polyethylenglykol 300, und
5 Gewichtsprozent in Glyzerol.

### Beispiel 8: Lösliches Pulver

25 Gewichtsteile Wirkstoff
1 Gewichtsteil Natriumlaurylsulfat,
3 Gewichtsteile kolloides Kieselgel, und
71 Gewichtsteile Harnstoff.

Die Bestandteile werden gemischt und bis zur Homogenität miteinander vermahlen.

Es können weitere biologisch aktive Substanzen oder Zusätze, die sich gegenüber den Wirkstoffen neutral verhalten und keinen schädlichen Einfluss auf das zu behandelnde Wirtstier haben, sowie Mineralsalze oder Vitamine den beschriebenen Kompositionen zugesetzt werden.

Analog zu den beschriebenen Formulierungen der Beispiele 1 bis 8 lassen sich auch weitere Präparationen mit Wirksubstanzen aus den weiter oben definierten Substanz-Klassen (a), (b) oder (c) herstellen.

### Beispiel 9: Flohbekämpfung durch orale Verabreichung des Wirkstoffs an das Wirtstier

Hunde mit der nachgewiesenen Fähigkeit, eine Flohpopulation permanent aufrechterhalten zu können, werden mit 100 Katzenflöhen [Ctenocephalides felis (Bouche)] infiziert. Die infizierten Hunde werden dann gemäss der nachfolgenden Tabelle A in 3 Gruppen zu je 2 Tieren eingeteilt. Die Hunde von 2 Gruppen werden 10 aufeinanderfolgende Tage oral mit 3 bzw. 5 mg/kg Körpergewicht/Tag der Wirksubstanz behandelt. Die Aktivsubstanz wird in Form einer wässrigen Suspension (mittels Spritze) verabreicht. Die dritte Gruppe von Tieren bleibt unbehandelt und dient als Kontrollgruppe.

Nach 3,8 und 10 Tagen werden die Floheier von dem Papier, das sich unter den Hundeboxen befindet, aufgesammelt. Die Eier werden gezählt und auf ein für die Anzucht von Larven geeignetes Medium gegeben und inkubiert. Die Anzahl der gebildeten Puppen und ausgewachsenen Flöhe wird gemäss Tabelle B bestimmt.

Die angegebenen Daten zeigen, dass 3 Tage nach Behandlung der Hunde mit 5 mg Wirkstoff/kg Körpergewicht/Tag sich keine erwachsenen Flöhe mehr aus Eiern von Flöhen zu bilden vermögen, die sich vom Blut des behandelten Hundes ernährt haben.

Diese Resultate verdeutlichen, dass ausgewachsene, weibliche Flöhe, die sich vom Blut eines mit 5 mg Wirkstoff/kg Körpergewicht/Tag behandelten Hundes ernähren, ausschliesslich solche Eier legen, die sich nicht mehr in ausgewachsene Flöhe zu verwandeln vermögen. Auf diese Weise wird der Lebenszyklus der Flöhe klar unterbrochen und die Ausbreitung der Flöhe im Sinne eines Wiederbefalls eindeutig verhindert. Nur so ist eine dauerhafte flohfreie Haltung von Hunden und Katzen gewährleistet.

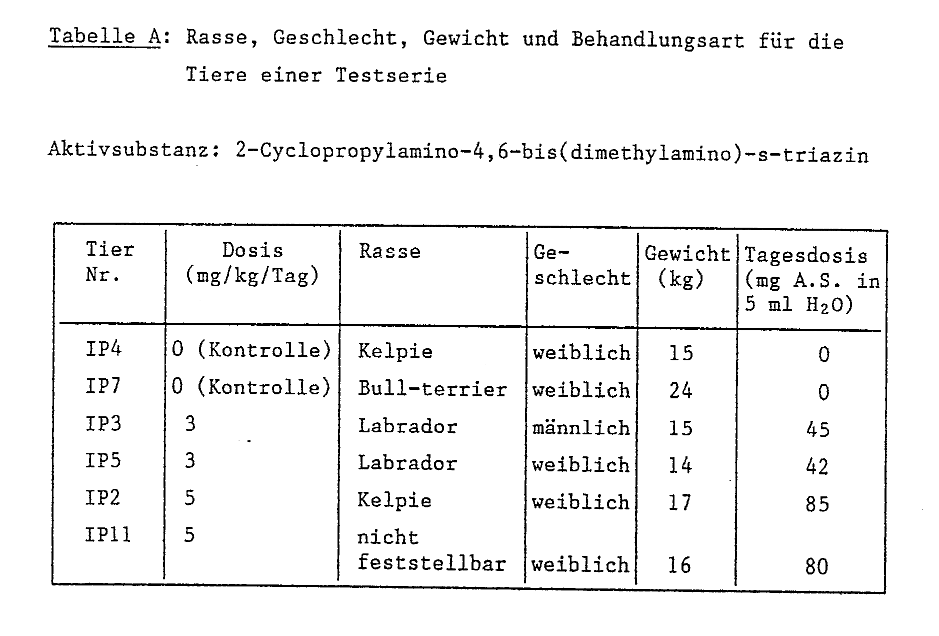

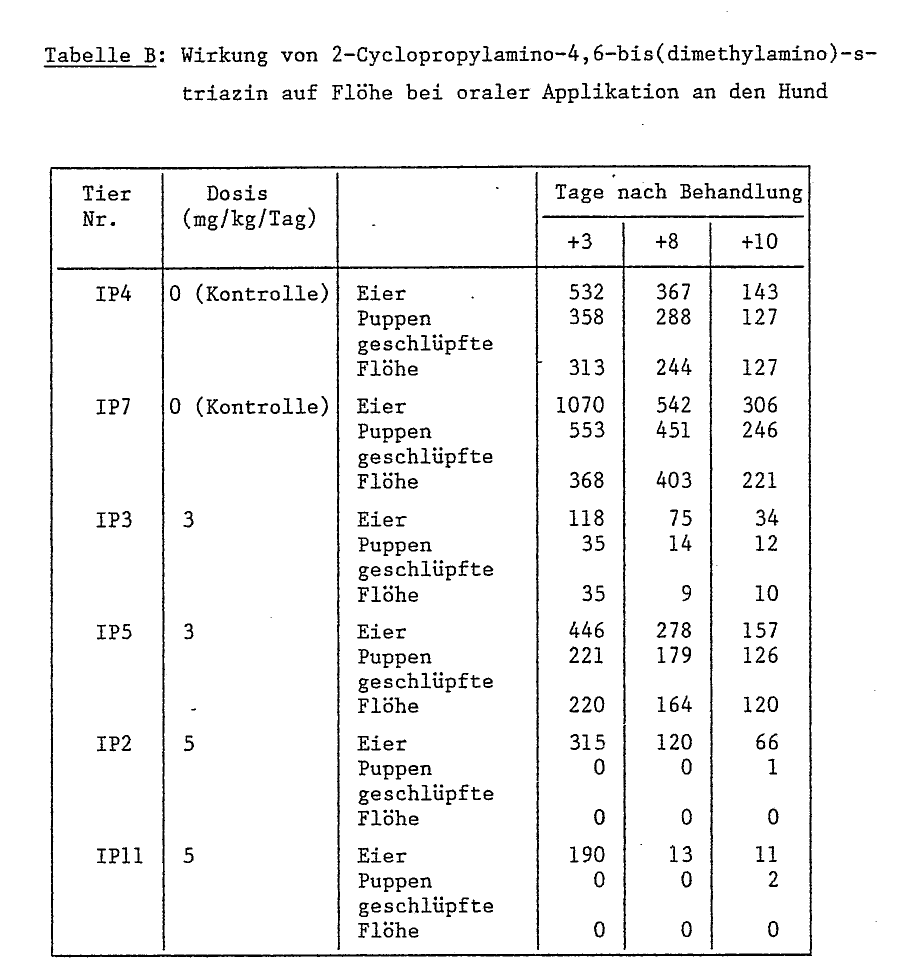

Identische Resultate wurden auch nach Verabreichung von Verbindung Nr. 2.5 festgestellt.

## Patentansprüche

1. Verfahren zur systemischen Verhinderung eines Wiederbefalls von Hunden und Katzen durch Flöhe, dadurch gekennzeichnet, dass man eine für Flöhe larvizid oder ovizid wirksame Menge einer das Flohwachstum hemmenden Substanz oral, parenteral oder in Form eines Implantates dem besagten Wirtstier verabreicht.

2. Verfahren nach Aspruch 1, dadurch gekennzeichnet, dass der zu verabreichende Wirkstoff
a) ein Juvenil-Hormon oder eine juvenil-hormon-artige Substanz,
b) ein das Flohwachstum hemmendes Benzoylharnstoff-Derivat oder
c) ein das Flohwachstum hemmendes Triazinderivat ist.

3. Verfahren nach Anspruch 2, dadurch gekennzeichnet, dass man als Wirkstoff ein Juvenil-Hormon oder eine wie ein Juvenil-Hormon wirkende Chemikalie einsetzt.

4.Verfahren nach Anspruch 2, dadurch gekennzeichnet, dass man als Juvenil-Hormon oder juvenil-hormon-artige Substanz eine Substanz einsetzt, ausgewählt aus den Vertretern der Gruppe

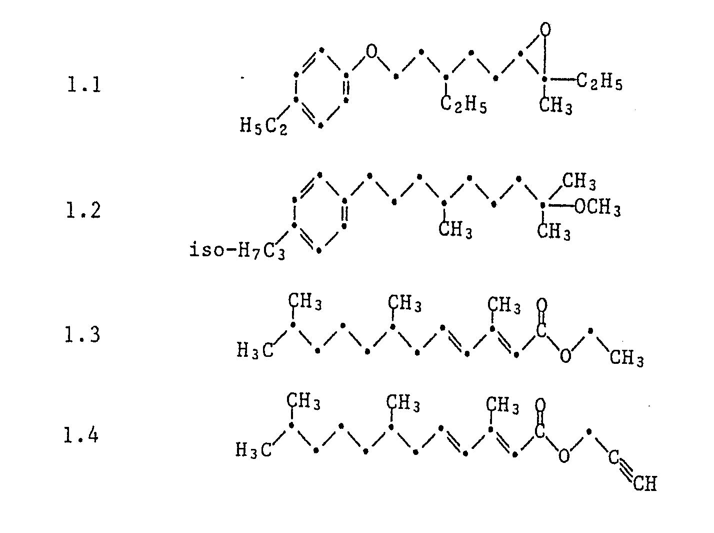

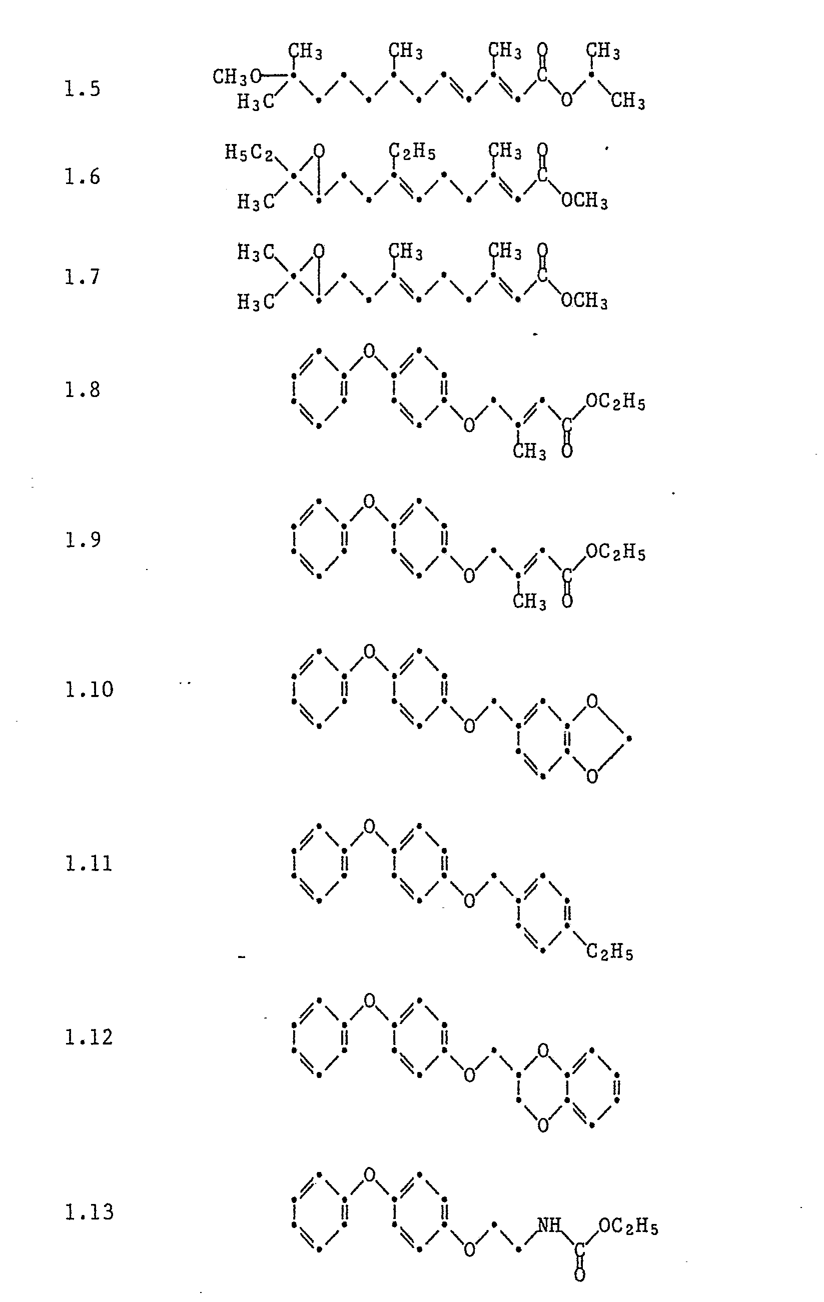

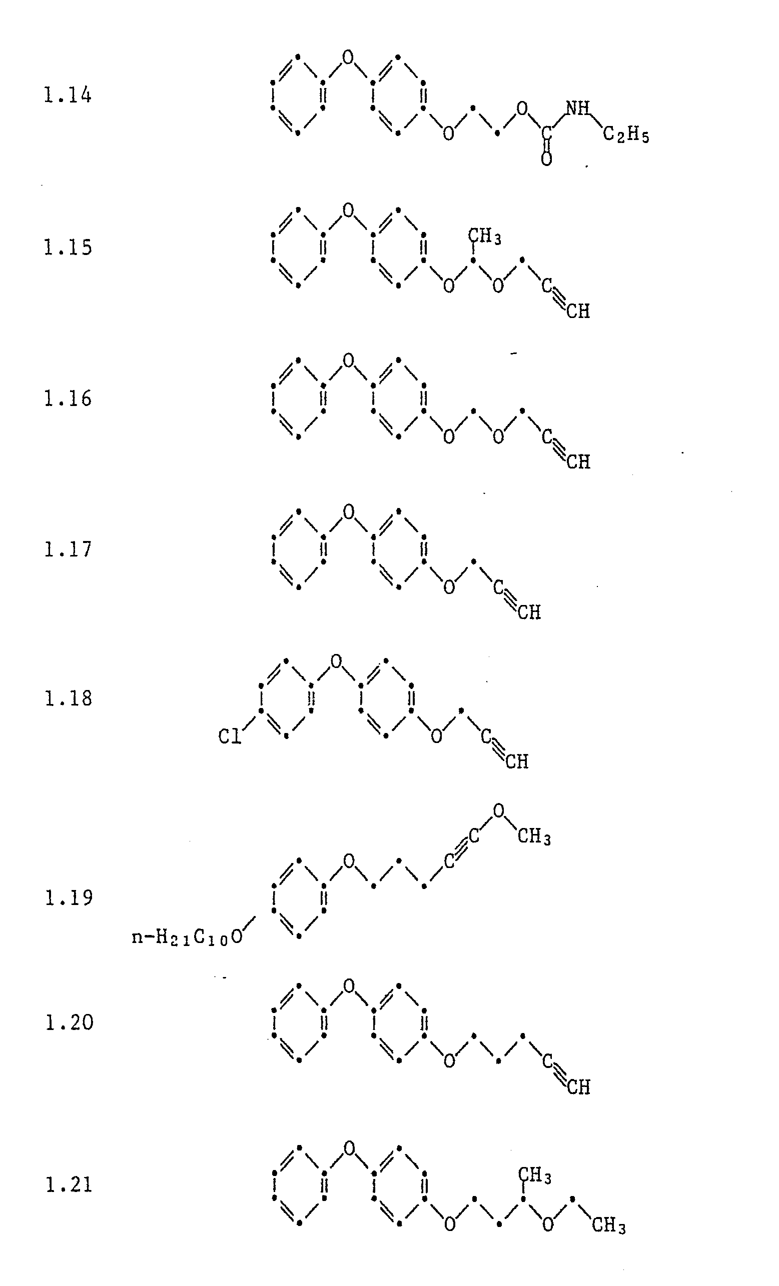

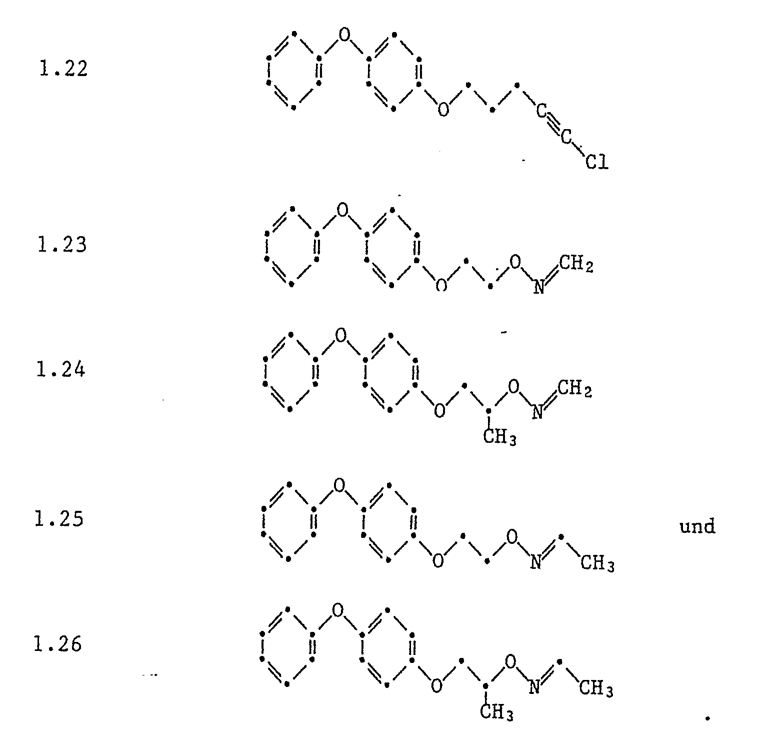

5. Verfahren nach Anspruch 2, dadurch gekennzeichnet, dass man als ein das Flohwachstum hemmendes Benzoylharnstoff-Derivat eine Verbindung der Formel I 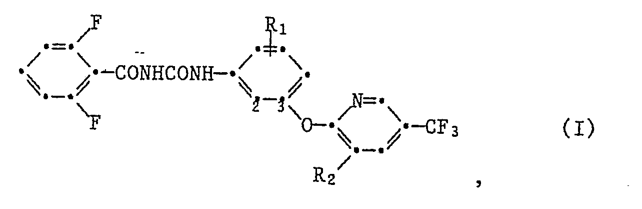 worin
R₁ für Wasserstoff, Halogen oder Methyl steht; und
R₂ Wasserstoff oder Halogen bedeutet, einsetzt.

6. Verfahren nach Anspruch 5, dadurch gekennzeichnet, dass das Benzoylharnstoff-Derivat ausgewählt ist aus den Vertretern der Gruppe

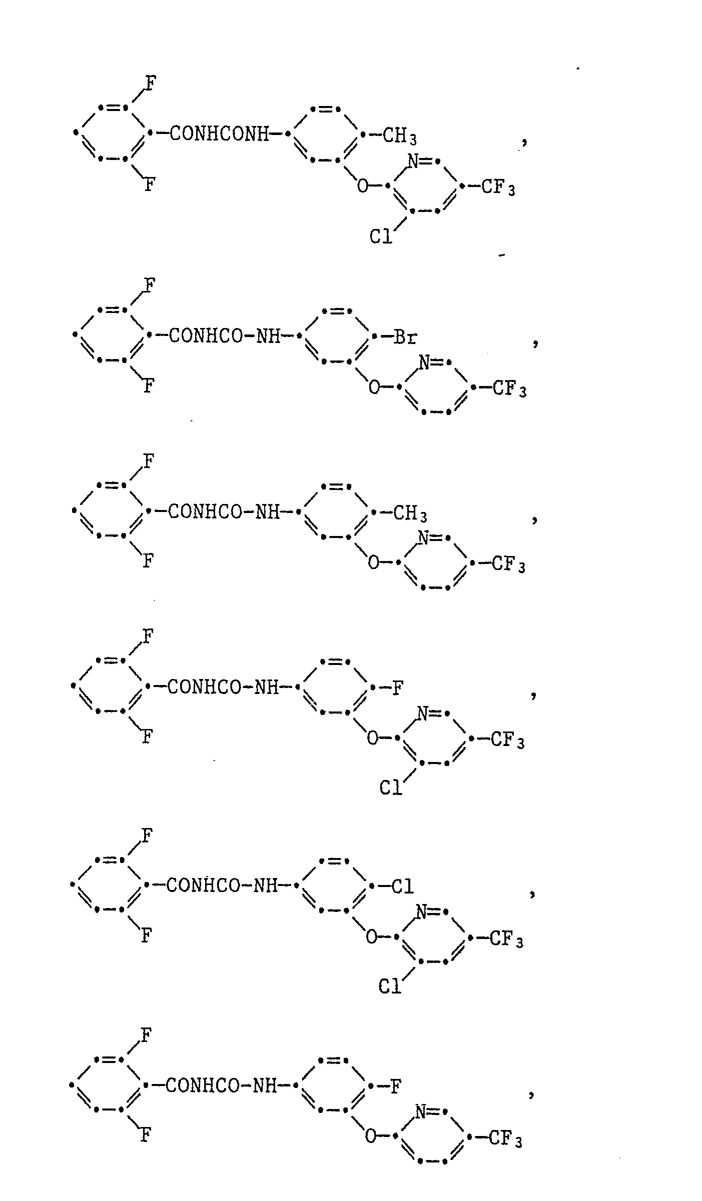

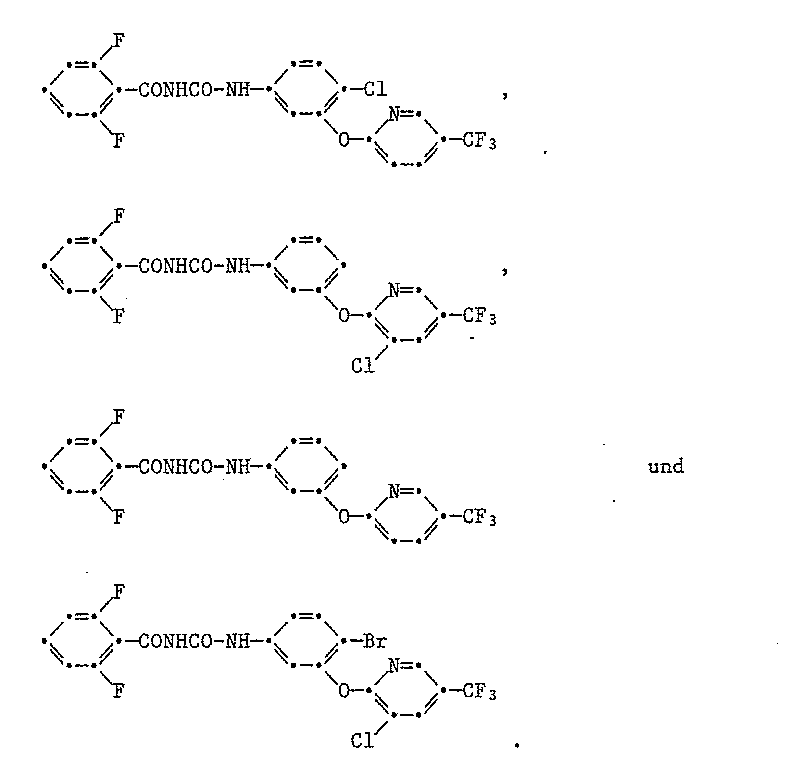

7. Verfahren nach Anspruch 2, dadurch gekennzeichnet, dass man als ein das Flohwachstum hemmendes Triazin-Derivat eine Verbindung der Formel X 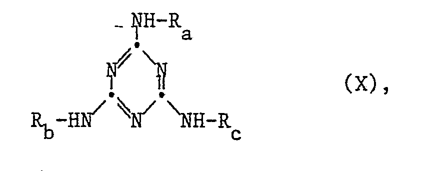 einsetzt, worin
Rₐ für Cycloalkyl oder Isopropyl;
R_{b} für Wasserstoff, Halogen, C₁-C₁₂-Alkylcarbonyl, Cyclopropylcarbonyl, C₁-C₁₂-Alkylcarbamoyl, C₁-C₁₂-Alkylthiocarbamoyl oder C₂-C₆-Alkenylcarbamoyl steht; und
R_{c} Wasserstoff, C₁-C₁₂-Alkyl, Cyclopropyl, C₂-C₆-Alkenyl, C₁-C₁₂-Alkylcarbonyl, Cyclopropylcarbonyl, C₁-C₁₂-Alkylcarbamoyl, C₁-C₁₂-Alkylthiocarbamoyl oder C₂-C₆-Alkenylcarbamoyl bedeutet, unter Einschluss der für Warmblüter untoxischen Säureadditionssalze.

8. Verfahren nach Anspruch 7, dadurch gekennzeichnet, dass das Triazin-Derivat ausgewählt ist aus der Gruppe

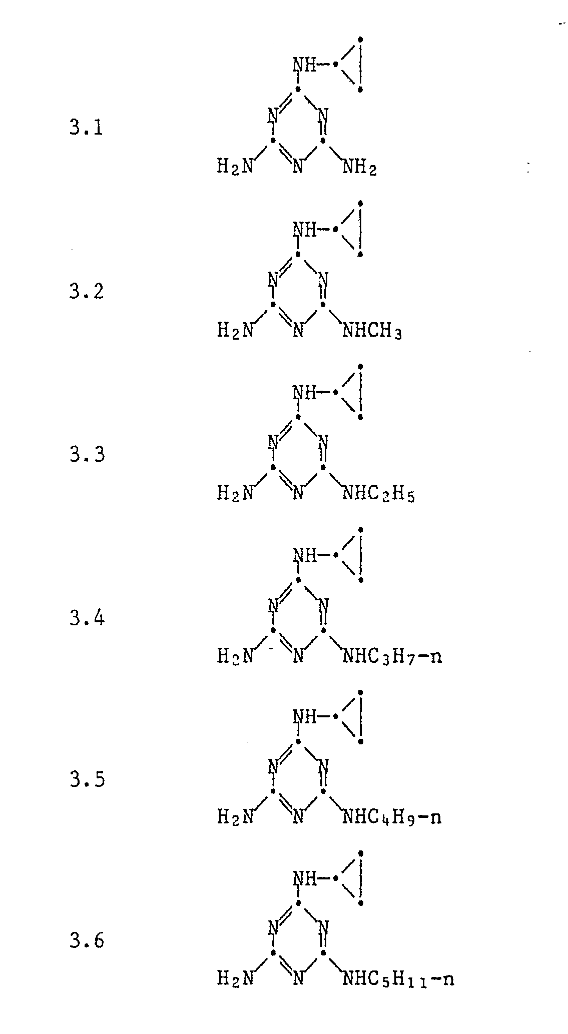

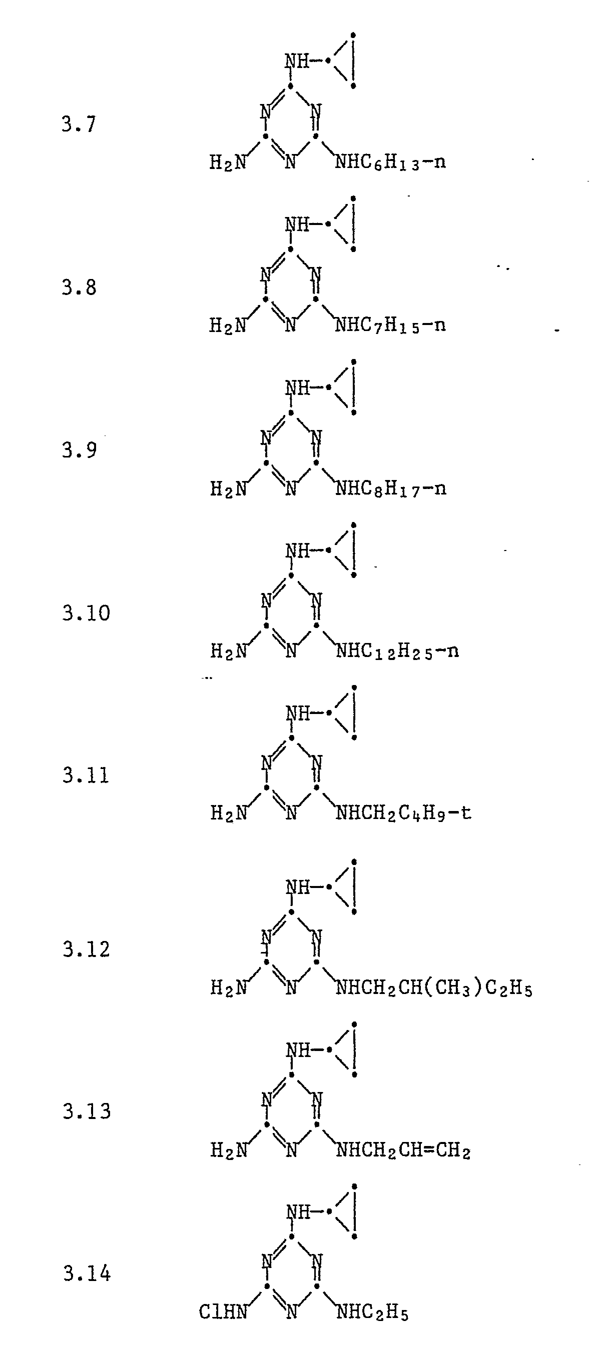

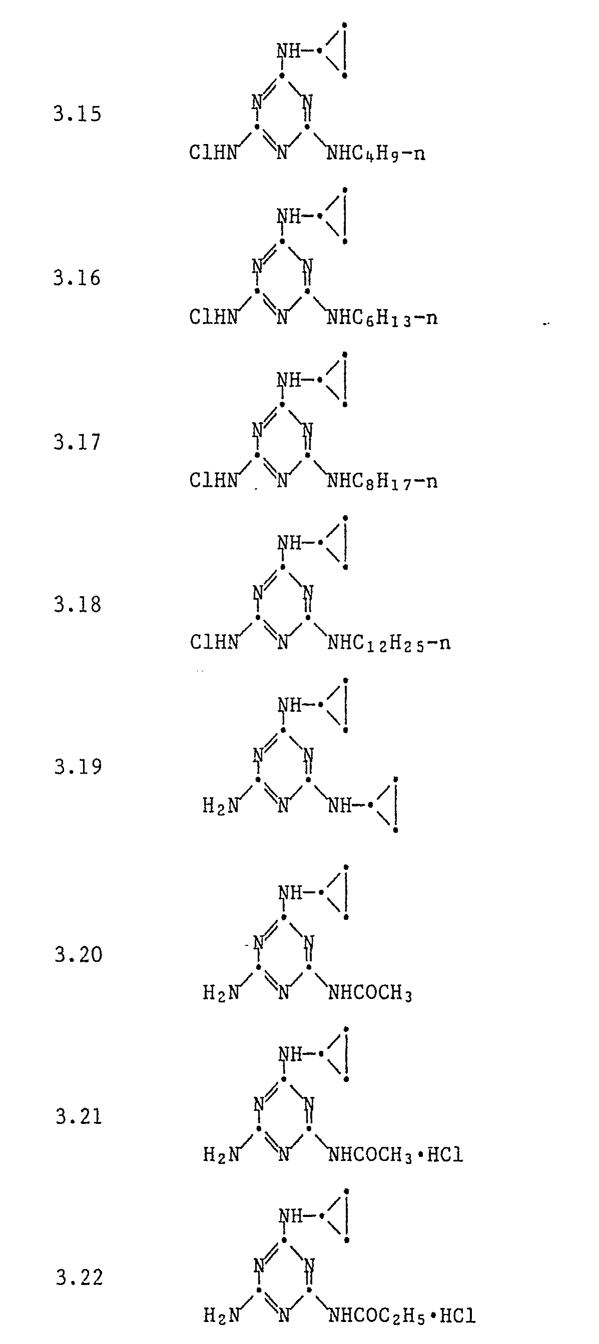

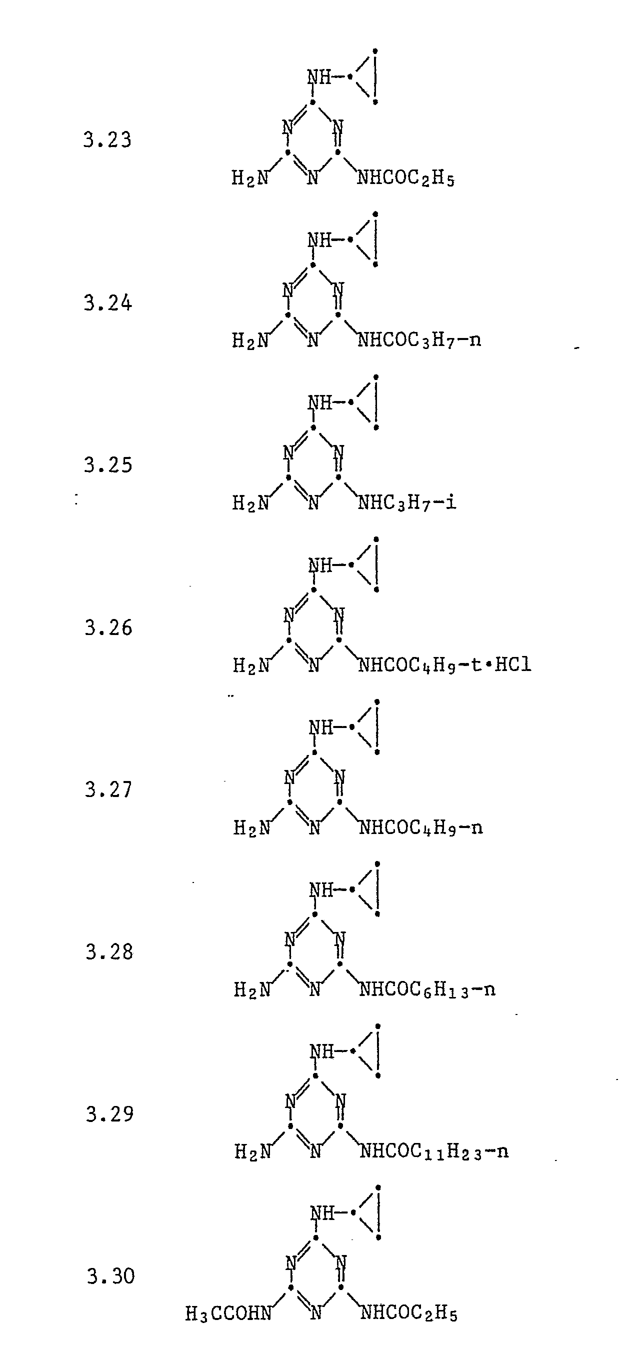

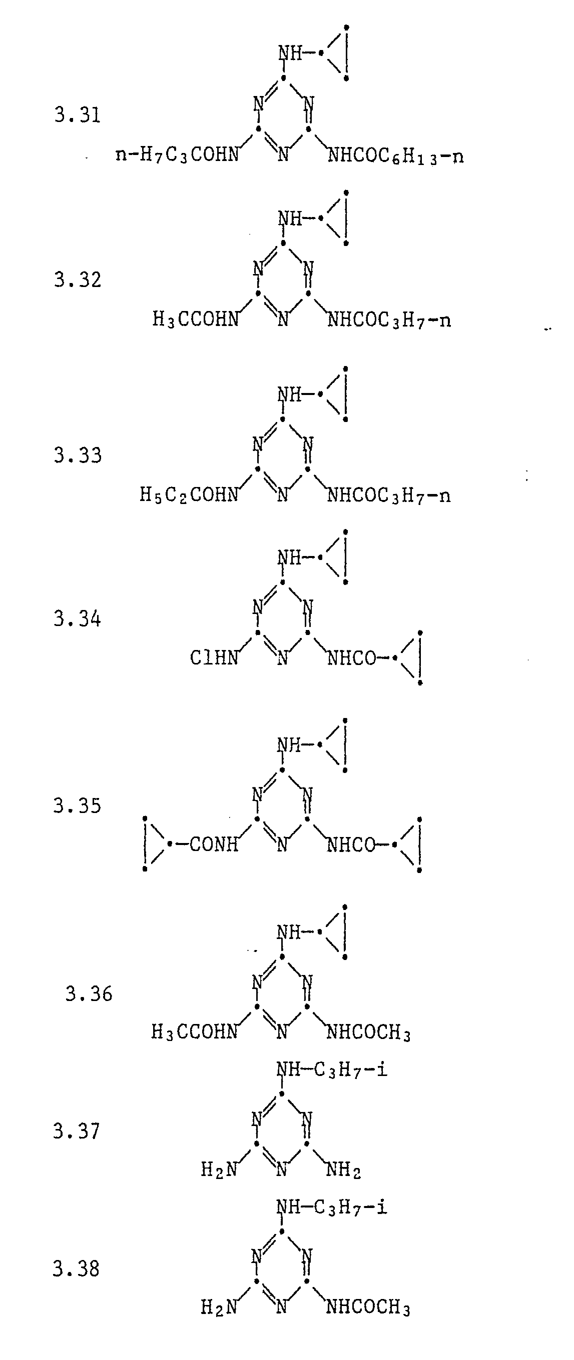

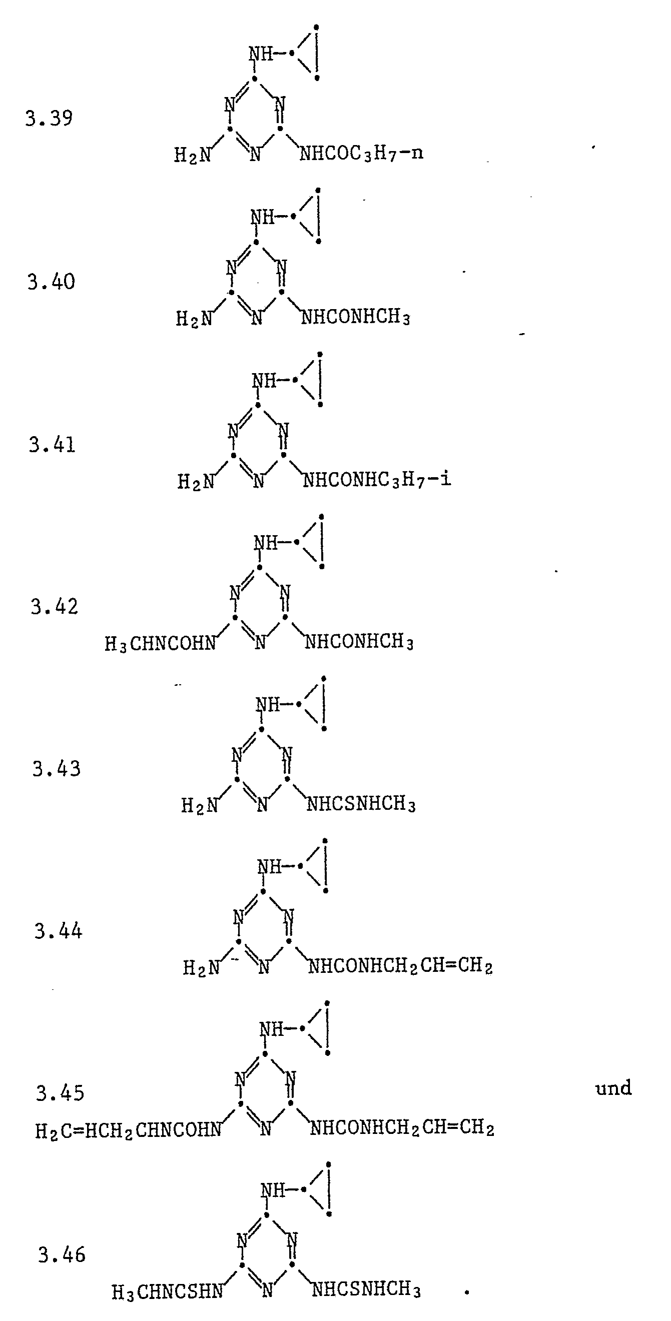

9. Verfahren nach Anspruch 8, dadurch gekennzeichnet, dass es sich bei dem ausgewählten Wirkstoff um Cyromazin handelt.

10. Verfahren nach Anspruch 1, dadurch gekennzeichnet, dass man die Aktivsubstanz dem Wirtstier in Aufwandmengen von etwa 0,01 mg/kg Körpergewicht bis etwa 800 mg/kg Körpergewicht verabreicht.

11. Verfahren nach Anspruch 10, dadurch gekennzeichnet, dass man die Aktivsubstanz dem Wirtstier in Aufwandmengen von etwa 0,5 mg/kg Körpergewicht bis etwa 200 mg/kg Körpergewicht verabreicht.

12. Verfahren nach Anspruch 10, dadurch gekennzeichnet, dass man die Aktivsubstanz dem Wirtstier in Aufwandmengen von etwa 1 mg/kg Körpergewicht bis etwa 30 mg/kg Körpergewicht verabreicht.

13. Verfahren nach Anspruch 10, dadurch gekennzeichnet, dass man den Wirkstoff der Katze oder dem Hund oral verabreicht.

14. Verfahren nach Anspruch 1, dadurch gekennzeichnet, dass man der Katze oder dem Hund regelmässig eine Dosis von 0,01 mg/kg Körpergewicht bis etwa 100 mg/kg Körpergewicht der Aktivsubstanz verabreicht.

15. Verfahren zur Verhütung der Vermehrung von Flöhen bei Hunden und Katzen, dadurch gekennzeichnet, dass man das Blut des Wirtstieres derart verändert, dass es eine das Flohwachstum hemmende Menge einer Substanz enthält, die ausgewählt ist aus der Gruppe der
a) Juvenil-Hormone oder juvenil-hormon-artig wirkenden Verbindungen;
b) das Flohwachstum hemmenden Benzoylharnstoff-Derivate; und
c) das Flohwachstum hemmenden Triazin-Derivate.

16. Verfahren nach Anspruch 15, dadurch gekennzeichnet, dass man eine wirksame Menge der Aktivsubstanz mit dem Futter dem Wirtstier verabreicht und diese durch die auf dem Wirtstier befindlichen Flöhe mit dem gesaugten Blut aufnehmen lässt.

17. Systemisch wirkendes Mittel zur Verhütung eines Wiederbefalls von Hunden und Katzen mit Flöhen, dadurch gekennzeichnet, dass es eine für Flöhe larvizid oder ovizid wirksame Menge einer das Flohwachstum hemmenden Substanz sowie inerte, für Hunde und Katzen untoxische und mit Hunde- und Katzenfutter verträgliche Zusätze enthält.

18. Mittel nach Anspruch 17, dadurch gekennzeichnet, dass es als Aktivsubstanz Cyromazin enthält.

19. Systemisch wirkendes Mittel zur Verhütung eines Wiederbefalls von Hunden und Katzen mit Flöhen, dadurch gekennzeichnet, dass es eine für Flöhe larvizid oder ovizid wirksame Menge einer das Flohwachstum hemmenden Aktivsubstanz und für Hunde und Katzen palatable Zusätze enthalt.

20. Mittel nach Anspruch 19, dadurch gekennzeichnet, dass es sich bei den palatablen Zusätzen um Hunde- oder Katzenfutter handelt.

21. Mittel nach Anspruch 19, enthaltend als Wirkstoff Cyromazin.
